# EUROPEAN PATENT APPLICATION

(11) **EP 4 509 146 A1**
(43) Date of publication of application: **19.02.2025**
(21) Application number: 23788453.1
(22) Date of filing: 13.02.2023
(51) Int. Cl.: A61L 27/18, A61L 27/36, A61F 2/02

(54) **ARTIFICIAL STRUCTURE FOR PREVENTING OR TREATING LYMPHEDEMA AND CONSTRUCTION METHOD THEREFOR**

(30) Priority: 12.04.2022 KR 20220045275
(71) Applicant: The Asan Foundation, Seoul 05505 (KR); University Of Ulsan Foundation For Industry Cooperation, Ulsan 44610 (KR)
(72) Inventor: JEON, Jae Yong, Seoul 05507 (KR); CHEON, Hwa Yeong, Seoul 05568 (KR)
(74) Representative: AWA Sweden AB
(86) International application number: PCT/KR2023/002064
(87) International publication number: WO 2023/200098

(57) **Abstract**

The present disclosure relates to an artificial structure for preventing or treating lymphedema and a construction method therefor. According to the artificial structure for preventing or treating lymphedema and the construction method therefor in accordance with an aspect, despite the effects of lymphadenectomy and radiation exposure, the flow of lymph fluid is maintained similarly to normal by the artificial structure, whereby the artificial structure has the effect of not only preventing the onset of lymphedema but also effectively being useful for treating diseases caused by interruption of lymph flow.

## Description

### Technical Field

The present disclosure relates to an artificial structure for preventing or treating lymphedema and a construction method therefor.

### Background Art

The lymphatic system consists of lymphatic vessels throughout the body in the form of specialized reticular connective tissue containing large amounts of lymphocytes, and lymph nodes and lymphatic organs that are connected to each other by the lymphatic vessels. Although the thymus among lymphatic organs consists of epithelial reticular tissue, in general, the outer structure of stroma in the lymphatic tissue consists of a reticular network with reticular fibers (fibroblasts) and reticular cells (fixed macrophages).

The lymphatic vessels are similar in structure to blood vessels, but are thin-walled and lined by large lymphatic ducts (lymphatic vessels) having many plates and include lymph nodes and lymph capillaries found in various parts of the body. The lymph nodes are concentrated in the face and neck, armpits, chest cavity, intestines, groin, elbows, and knees. In general, superficial lymphatic vessels extend along the vein, whereas deep lymphatic vessels extend along the artery. The lymphatic vessels supply lymph throughout the body and return proteins leaking out of capillaries to the cardiovascular system. In addition, the lymphatic vessels transport fat from the stomach into the blood. In cancer patients, the lymphatic tissue has a function of monitoring and defending against foreign cells, bacteria, and cancer cells. T cells constituting the lymphocytes play a role in directly or indirectly destroying invaders thereof by releasing various substances such as cytokines. B cells constituting the lymphocytes differentiate into plasmacytes that secrete antibodies against foreign antigens to help eliminate the foreign antigens. The lymph nodes act as a filter for foreign substances transported by the lymph by means of reticular fibrous tissue of the lymph nodes. Then, macrophages destroy the foreign substances by phagocytosis. The lymph nodes produce lymphocytes, and some of the lymphocytes are transported to other parts of the body as part of the immune defense system in lymph. The spleen, thymus, and tonsils are included in the lymphatic organs, which produce B-cells, T-cells, and lymphocytes, together with antibodies, respectively, to complete the immune defenses of the lymphatic system.

Lymphedema refers to a subcutaneous pooling condition of lymph fluid due to obstruction of the lymphatic vessels, and is a result of obstruction of lymphatic drainage by degenerative products upon apoptosis. Lymphedema can be divided into primary lymphedema caused by congenital and genetic abnormalities and secondary lymphedema acquired due to surgery, radiotherapy, etc. In particular, the secondary lymphedema often appears as a sequela after surgery and radiotherapy for breast cancer and uterine cancer. Therefore, patients with breast cancer or uterine cancer who have had surgery or radiotherapy are generally considered as potential patients having the secondary lymphedema.

However, essentially repeated radiotherapy causes continued and increasing obstruction of lymphatic drainage in exposed regions, resulting in prolonged lymphedema and increasingly serious side effects. Treatments for such lymphedema mostly rely on conservative treatments such compression bandages, physical therapy, and massage. The development of drugs for the treatment of lymphedema is currently at an insufficient level, and therapies, such as lymphatic massage, compression therapy, exercise, skin care, and the like that are commonly used so far cannot be fundamental and efficient solutions.

In addition, the existing artificial structures that are surgically introduced to provide the flow of lymph have a very low utilization rate because construction methods for these structures are complicated and require microsurgery, and thus there is a need for an improved artificial structure.

### Disclosure

### Technical Problem

One aspect is to provide an artificial structure including: a first sheet; and a second sheet arranged on the first sheet and including at least two patterned channels, each of which includes an inlet and an outlet and is patterned on a first surface facing the first sheet, wherein the at least two channels each have a one-way channel structure that maintains a one-way flow of lymph from the inlet toward the outlet.

Another aspect is to provide a construction method for an artificial structure, wherein the artificial structure includes: a first sheet; and a second sheet arranged on the first sheet and including at least two channels, each of which includes an inlet and an outlet and is patterned on a first surface facing the first sheet, wherein the at least two channels each have a one-way channel structure that maintains a one-way flow of lymph from the inlet toward the outlet, the construction method including: preparing the first sheet and the second sheet that are prepared from a polymer; and adhering the first sheet and the second sheet to position patterning on the first side of the second sheet to face inwardly.

### Technical Solution

One aspect provides an artificial structure including: a first sheet; and a second sheet arranged on the first sheet and including at least two channels, each of which includes an inlet and an outlet and is patterned on a first surface facing the first sheet, wherein the at least two channels each have a one-way channel structure that maintains a one-way flow of lymph from the inlet toward the outlet.

In an embodiment, the first sheet and the second sheet may be prepared from a biocompatible polymer.

In an embodiment, the biocompatible polymer may be any one selected from the group consisting of polylactic acid (PLA), polyglycolic acid (PGA), poly-ε-caprolactone (PCL), poly-L-lactic acid (PLLA), a poly(lactide-co-glycolic acid) (PLGA) copolymer, a poly(lactide-caprolactone (PLCL) copolymer, biodegradable polycarbonate, and a copolymer thereof.

In an embodiment, the biocompatible polymer may be hydrophobic.

In an embodiment, the artificial structure may constrict the at least two channels by the skeletal muscle pressure at an implanted site.

In an embodiment, the artificial structure may form a lymphatic network.

In an embodiment, the at least two channels may each have a diameter of 200 to 1000 µm.

In an embodiment, the at least two channel may each include: a primary channel connecting the inlet to the outlet and providing a direction of a main flow of lymph; and at least two anti-backflow channels, each of which is connected to the primary channel and has a structure that prevents a backflow.

In an embodiment, the primary channel may have a zig-zagged linear structure, and the at least two anti-backflow channels may each have a curved structure.

In an embodiment, the at least two anti-backflow channels may be staggered at regular intervals with respect to the primary channel.

In an embodiment, the artificial structure may be implanted in any one of a branchial lymph node (BLN), a superficial inguinal node, a deep inguinal node, and a popliteal lymph node.

In an embodiment, the artificial structure may be implanted in a BLN.

Another aspect provides a construction method for an artificial structure, wherein the artificial structure includes: a first sheet; and a second sheet arranged on the first sheet and including at least two channels, each of which includes an inlet and an outlet and is patterned on a first surface facing the first sheet, wherein the at least two channels each have a one-way channel structure that maintains a one-way flow of lymph from the inlet toward the outlet, the construction method including: preparing the first sheet and the second sheet that are prepared from a polymer; and adhering the first sheet and the second sheet to position patterning on the first side of the second sheet to face inwardly.

In an embodiment, the polymer may be polydimethylsiloxane (PDMS).

In an embodiment, the artificial structure may be prepared from a biocompatible polymer.

In an embodiment, the biocompatible polymer may be any one selected from the group consisting of PLA, PGA, PCL, PLLA, a PLGA copolymer, a PLCL copolymer, biodegradable polycarbonate, and a copolymer thereof.

In an embodiment, the biocompatible polymer may be hydrophobic.

### Advantageous Effects

According to an artificial structure for preventing or treating lymphedema and a construction method therefor in accordance with an aspect, despite the effects of lymphadenectomy and radiation exposure, the flow of lymph fluid is maintained similarly to normal by the artificial structure, whereby the artificial structure has the effect of not only preventing the onset of lymphedema but also effectively being useful for treating diseases caused by interruption of lymph flow.

### Description of Drawings

FIG. 1 is a diagram illustrating a perspective view of an artificial structure for preventing or treating lymphedema.
FIG. 2 is a diagram showing a channel structure that implements an anti-backflow function of the artificial structure.
FIG. 3 is diagram illustrating a construction method for the artificial structure for preventing or treating lymphedema.
FIG. 4 is a photograph showing fluid flow through a channel by injecting a fluorescent substance and a staining substance into the artificial structure, according to an embodiment.
FIG. 5 schematically illustrates a lymphadenectomy situation imitated through folding suture in a rat, and an implantation of the artificial structure of the present disclosure, according to an embodiment.
FIG. 6 shows a control group in which a lymphadenectomy situation is imitated according to an embodiment.
FIG. 7 shows an experimental group in which the artificial structure of the present disclosure is implanted after a lymphadenectomy situation is imitated, according to an embodiment.
FIG. 8 is a graph showing the results of comparing volume changes between an experimental group and a control group, in evaluating efficacy of the artificial structure, according to an embodiment.
FIG. 9 is a photograph confirming the flow of lymph by fluorescein angiography, in evaluating efficacy of the artificial structure, according to an embodiment.
FIG. 10 is a photograph confirming the flow of lymph by using a staining substance, Evans Blue, 8 weeks after implantation of the artificial structure, in evaluating efficacy of the artificial structure, according to an embodiment.
FIG. 11 is a graph showing the results of comparing drainage patterns of lymph between an experimental group and a control group, in evaluating efficacy of an artificial structure, according to an embodiment.
FIG. 12 is a photograph confirming lymphangiogenesis through cross-sectional staining of the artificial structure 8 weeks after implantation of the artificial structure, in evaluating efficacy of the artificial structure, according to an embodiment.
FIG. 13 is a photograph confirming the flow of lymph by fluorescein angiography 4 weeks after removal of the artificial structure, in evaluating efficacy of the artificial structure, according to an embodiment.

### Best Mode

Embodiments may undergo various modifications, and thus specific embodiments will be illustrated in the drawings and described in detail in the detailed description. Effects and characteristics of embodiments, and methods of accomplishing the same will be apparent when referring to embodiments described with reference to the drawings. The embodiments may, however, be embodied in many different forms and should not be construed as limited to the embodiments set forth herein. Hereinafter, embodiments will be described in detail with reference to the accompanying drawings. The same or corresponding components will be denoted by the same reference numerals, and thus redundant description thereof will be omitted. It will be understood that although the terms "first," "second," etc. may be used herein to describe various components, these components should not be limited by these terms. These components are only used to distinguish one component from another. It will be further understood that the terms "comprises" and/or "comprising" used herein specify the presence of stated features or elements, but do not preclude the presence or addition of one or more other features or elements. Sizes of elements in the drawings may be exaggerated for convenience of explanation. In other words, because sizes and thicknesses of components in the drawings are arbitrarily illustrated for convenience of explanation, the following embodiments are not limited thereto.

One aspect of the present disclosure provides an artificial structure including: a first sheet; and a second sheet arranged on the first sheet and including at least two channels, each of which includes an inlet and an outlet and is patterned on a first surface facing the first sheet, wherein the at least two channels each have a one-way channel structure that maintains a one-way flow of lymph from the inlet toward the outlet.

In the present specification, the term "edema" refers to the presence of excessive pooling of liquid, such as lymph fluid and the like, within tissue. The edema may be lymphedema, but is not limited thereto as long as it is a disease characterized by the presence of excessive pooling of liquid within tissue.

In the present specification, the term "prevention" refers to, for example, blocking of the appearance of diseases or disease-related symptoms, in an asymptomatic subject at risk of developing a disease.

In the present specification, the term "treatment" refers to a therapeutic intervention that ameliorates signs or symptoms of disease or pathological conditions after the onset of the disease or pathological conditions. The "amelioration" with respect to the signs or symptoms of disease or pathologic conditions refers to an observable beneficial effect of treatment. The beneficial effect may be demonstrated by, in a subject susceptible to a disease, a delayed onset of clinical symptoms of disease, a reduction in severity of some of all clinical symptoms of a disease, a slower progression of a disease, an improvement in overall health or well-being of a subject, or any other variable known in the art to be specific to a particular disease.

Meanwhile, "treatment" and "prevention" should be considered in the broadest sense. In this regard, "treatment" does not necessarily imply that a subject is treated until the subject is fully recovered, and "prevention" also does not necessarily mean that a subject will not ultimately develop a disease. Therefore, treatment and prevention include relieving symptoms of a certain disease and preventing or reducing the risk of developing a certain condition.

In the present specification, the term "artificial structure for preventing or treating lymphedema" refers to a biocompatible artificial structure available for in vivo implantation, and is a two-dimensional sheet-type structure. The artificial structure may serve to maintain the flow of lymph against factors that interfere with the flow of lymph after lymphadenectomy. The factors that interfere with the flow of lymph may include constriction of lymphatic vessels, tissue fibrosis, etc.

In the present specification, the term "one-way channel structure" refers to a channel structure configured to maintain the flow of lymph in one direction. Specifically, such a channel structure may include a structure that implements a valve function by maintaining the flow of lymph passing through a channel in one direction from an inlet toward an outlet.

In the present specification, the expression "implementation of a valve function" refers to imitation of the same or similar valve function that allows fluid, such as lymph fluid and the like, to flow in one direction only. Specifically, this expression may refer to implementation of a function that blocks a backflow of lymph by a main flow and maintains the flow only in a direction of the main flow.

In an embodiment, the artificial structure may be prepared from a polymer.

In an embodiment, the artificial structure may be prepared from a biocompatible polymer. In the present specification, the term "biocompatible polymer" refers to a polymer that has tissue compatibility and blood compatibility that do not destroy or clot blood upon contact with biological tissue or blood, may include a polymeric material that readily adapts to the in vivo environment and is therefore not rejected. Specifically, the biocompatible polymer may include materials used in a variety of applications, ranging from prostheses, implants, and artificial organs that can be inserted or implanted into the body, and may not cause inflammation around the implant and may be absorbed into the body after implantation if necessary. The artificial structure may be prepared from polydimethylsiloxane (PDMS), polyethersulfone (PES), poly(3,4-ethylenedioxythiophene), poly(styrenesulfonate), polyimide, polyurethane, polyester, perfluoropolyether (PFPE), polycarbonate, or a combination thereof.

The biocompatible polymer may be a material that not only has excellent physical stability to maintain its shape after insertion into the body, but also has excellent biodegradability. Specifically, the biocompatible polymer may include any one selected from the group consisting of polylactic acid (PLA), polyglycolic acid (PGA), poly-ε-caprolactone (PCL), poly-L-lactic acid (PLLA), a poly(lactide-co-glycolic acid) (PLGA) copolymer, a poly(lactide-caprolactone (PLCL) copolymer, biodegradable polycarbonate, and a copolymer thereof.

In an embodiment, the channel may form a lymphatic network. Specifically, the channel may form an artificial lymphatic network. In addition, the channel may have an inlet and an outlet so that the channel may serve as a pathway for the flow of cells, tissue fluid, lymph, immune cells, etc., when implanted in the body.

In the present specification, the expression "formation of a lymphatic network" refers to an artificial structure designed to be implanted in or around a surgical site to treat impaired flow of lymph from various causes, such as lymphadenectomy or radiation exposure, or to artificial formation of an environment identical or similar to that of a lymph node. Specifically, the formation of a lymphatic network may include induction of lymphangiogenesis at or around the implanted surgical site. The induction of lymphangiogenesis may include maintaining a lymphangiogenesis-friendly environment after lymphadenectomy, such as preventing fibrosis of surrounding tissue and maintaining space for lymphangiogenesis.

In an embodiment, the artificial structure in which the lymphangiogenesis is induced may include any one selected from the group consisting of endothelial cells, vascular endothelial cells, perivascular cells, fibroblasts, stem cells, lymphocytes, B cells, NK cells, T cells, dendritic cells, lymphocyte endothelial cells, and a combination thereof, inside the artificial structure.

In an embodiment, the artificial structure may be to constrict the channel by skeletal muscle pressure. Specifically, the channel may be a 2D thin film structure with high elasticity, and may be therefore constricted by skeletal muscle pressure.

In an embodiment, the channel may have a diameter ranging from 200 to 1,000 µm. Specifically, the channel may have a diameter of at least 50 µm, 50 to 200 µm, or 200 to 1,000 µm, but the diameter is not limited thereto.

In an embodiment, the artificial structure may be cut to the same size as a surgical resection site, and implanted thereto, thereby performing roles of artificial lymph in vivo. Specifically, for the purpose of preventing secondary lymphedema by lymphadenectomy or radiation exposure, the artificial structure may be cut to fit the size of a resection site during lymphadenectomy and then implemented to perform roles of lymph, such as maintaining the flow of lymph in vivo.

In an embodiment, the artificial structure may have a multi-channel structure including at least two channels, and when implemented in vivo, the artificial structure may facilitate connection with tissues of the living body by a suturing method or the like. Specifically, such a multi-channel structure may utilize space between channels to enable fixing between the artificial structure and the biological tissue. Accordingly, the artificial structure may increase the possibility that the artificial structure will be connected with the living tissue when implanted in vivo.

In an embodiment, when the artificial structure is used for the purpose of treating or ameliorating a lymphedema-related condition, the artificial structure may be cut in a longer length than the artificial structure used for the purpose of preventing the same condition. Specifically, the lymphedema-related condition may be relevant to treating a disease and condition associated with lymphatic vasculature. Non-limiting examples of the disease and condition associated with the lymphatic vasculature may include primary lymphedema (e.g., Milroy disease, Meige lymphedema, and lymphedematarda), secondary lymphedema, and lymphedema such as lipedema.

Another aspect provides a construction method for an artificial structure including: a first sheet; and a second sheet arranged on the first sheet and including at least two channels, each of which includes an inlet and an outlet and is patterned on a first surface facing the first sheet, wherein the at least two channels each have a one-way channel structure that maintains a one-way flow of lymph from the inlet toward the outlet, the construction method including: (a) preparing the first sheet and the second sheet that are prepared from a polymer; and (b) adhering the first sheet and the second sheet to position patterning on the first side of the second sheet to face inwardly.

The processes (a) and (b) are not intended to imply any order of processes.

In an embodiment, the method may include, before the process (a), manufacturing a mold of a channel structure. Specifically, the manufacturing of a mold may include laser-patterning on the mold.

In an embodiment, the laser-patterning of the mold may implement a channel structure having a valve function. Specifically, by means of the laser-patterning, a channel structure may be implemented, the channel structure including a primary channel having a zig-zagged linear structure and an anti-backflow channel staggered at regular intervals with respect to the primary channel.

In an embodiment, the process (b) may include, after positioning the first sheet and the second sheet to position patterning on the first side of the second sheet to face inwardly, adhering the two sheets. Specifically, through the adhering, the channel structure may be included within the artificial structure, and so that the channels may be formed to allow lymph fluid or the like to pass through.

In an embodiment, the method may include, after the process (b), performing oxygen plasma treatment or other processes of introducing hydrophilicity to the artificial structure besides the oxygen plasma treatment. In an embodiment of the present disclosure, the method may include, after the adhering of the first sheet and the second sheet, performing oxygen plasma treatment at 80 to 90 °C for 15 to 30 minutes.

In an embodiment, a ratio of the polymer to the curing agent may be at least 9:1 to 10:1, at least 10:1 to 11:1, or at least 11:1 to 12:1, to manufacture a sheet. In an embodiment of the present disclosure, PDMS and the curing agent may be mixed at a ratio of 10:1 to implement the artificial structure.

In an embodiment, a thickness of the first sheet or a thickness of the second sheet is not particularly limited, but may be 500 µm.

In an embodiment, the biocompatible polymer may be highly hydrophobic.

### Mode for Invention

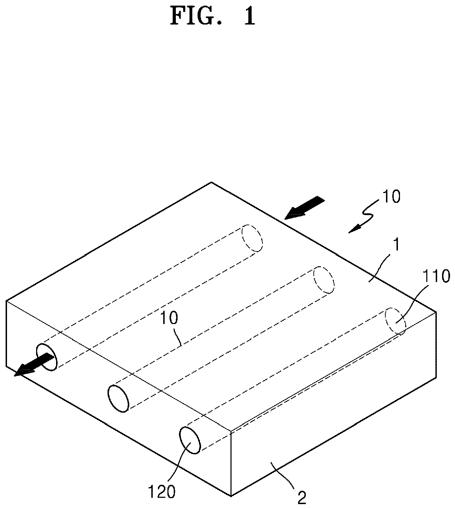

Referring to FIG. 1, provided is an artificial structure 100 for preventing or treating lymphedema, the artificial structure 100 including: an inlet 110 to which lymph is injected; at least two channels 10 through which lymph passes; and an outlet 120 from which the lymph that has passed through the channel 10 is discharged. The artificial structure 100 includes: a first sheet 1; and at least two sheets 2 each of which includes the inlet 110 and the outlet 120 and is patterned on a first surface facing the first sheet 1. In addition, the channel 20 may have a one-way channel structure that maintains the flow of lymph in one direction from the inlet 110 toward the outlet 120.

The first sheet 1 and the second sheet 2 are basic scaffolds that allow the artificial structure 100 to include at least two channels 10.

The channel 10 refers to a hollow passage space inside the artificial structure 100, and may serve as a pathway for immune cells, lymph, tissue fluid, etc., to flow when implanted in vivo, thereby forming a lymphatic network.

The structure of the channel 10 may be in any shape as long as the channel 10 allows lymph passing through the channel to flow in one direction.

The structure of the channel 10 may connect the inlet 110 to the outlet 120, and may include: a primary channel 210 that provides a direction of the main flow of lymph; and at least two anti-backflow channels 220 each having a structure that prevents the backflow. Specifically, the channel 10 may have a structure in which the primary channel 210 has a zig-zagged linear structure and the anti-backflow channel 220 has a curved structure. Here, the at least two anti-backflow channels 220 may be staggered at regular intervals with respect to the primary channel 210.

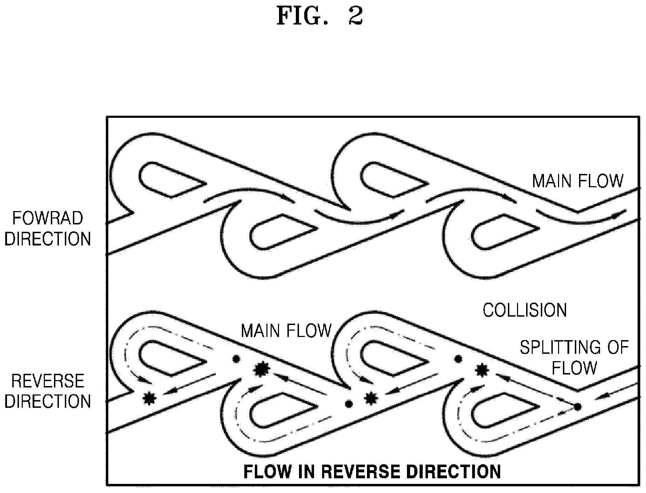

Referring to FIG. 2, the structure of the channel 10 of the artificial structure 100 connects the inlet 110 to the outlet 120, and includes the primary channel 210 that provides a direction of the main flow of lymph and at least two anti-backflow channel 220 connected to the primary channel 210. Regarding the one-way channel structure that prevents backflow of lymph and maintains the flow of lymph in one direction, specifically, the channel 10 may have a structure in which the primary channel 210 has a zig-zagged linear structure and the anti-backflow channel 220 has a curved structure, wherein the anti-backflow channel 220 may be staggered at regular intervals with respect to the primary channel 210.

The primary channel 210 connects the inlet 110 to the outlet 120 of the artificial structure, and the structure of the primary channel 210 is not limited to a zig-zagged linear structure or a straight structure, as long as the structure can provide a direction of the main flow of lymph.

The structure of the anti-backflow channel 220 is not limited to a curved structure, as long as the structure can maintain a direction of the main flow and interfere with the flow in the reverse direction.

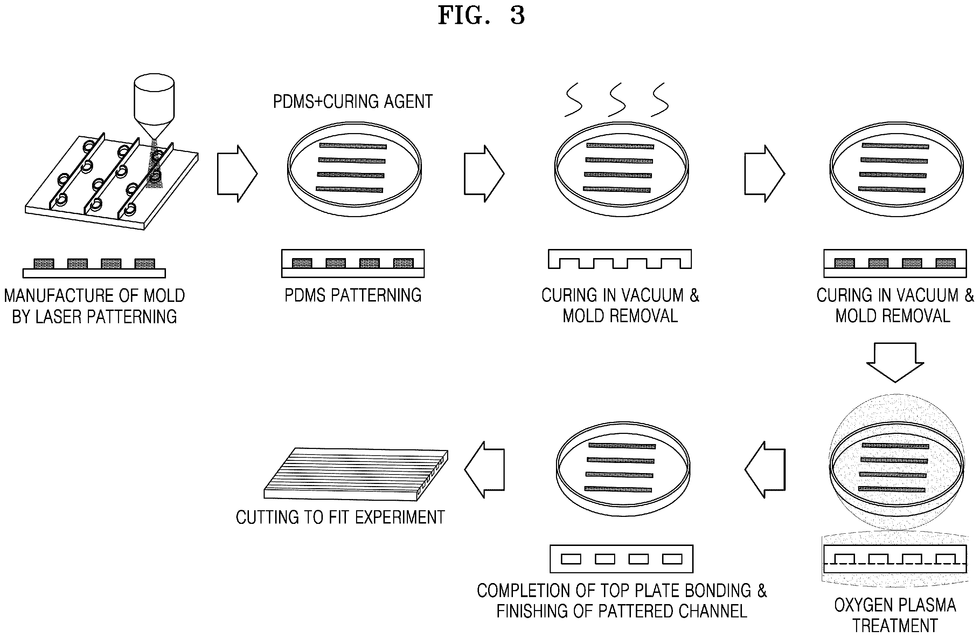

Referring to FIG. 3, as a construction method for the artificial structure 100, the method includes manufacturing a mold of the channel 10 through laser patterning. On a mold thus manufactured, a mixture of PDMS and a curing agent mixed at a mass ratio of 12:1 was poured, the resultant mold was subjected to a curing process in a vacuum, and then, the mold was removed. After adhering the PDMS top plate, oxygen plasma was treated at 80 °C to 90 °C for 15 minutes to 30 minutes, thereby completing the manufacture of the artificial structure 100 having at least two channels 10 via the top plate. The artificial structure 100 thus manufactured may be cut to the size of the resection site during lymphadenectomy, for use in the prevention of secondary lymphedema.

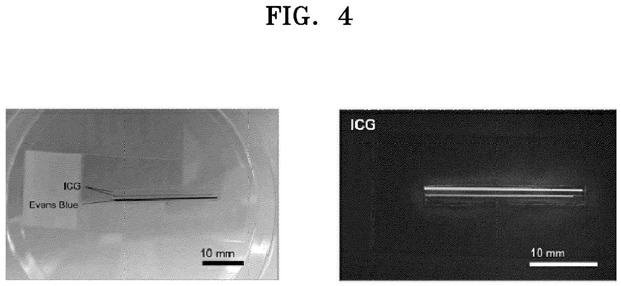

Referring to FIG. 4, the artificial structure 100 is in the form of a two-dimensional sheet including at least two channels 10, and may allow addition of a fluorescent material and a staining substance thereto and passage thereof through the channels 10.

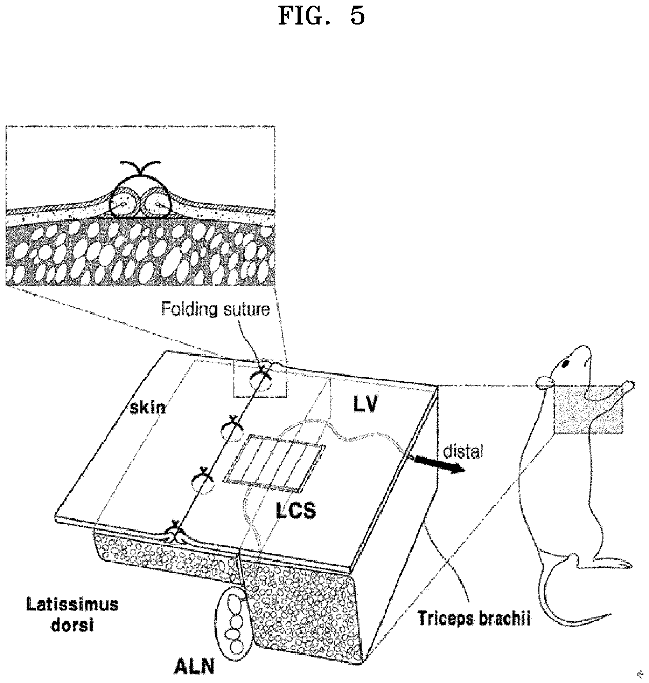

Referring to FIG. 5, to determine the effects of the artificial structure 100, a method of implanting an artificial structure in an animal model is illustrated schematically. After the BLN where lymph flow of the upper limb collects is excised, an artificial structure (i.e., a lymphatic channel sheet (LSC)) is implanted, and radiation is applied thereto to stimulate lymphadenectomy and radiotherapy that are performed on the human body. Considering that the existing BLN of the upper limb collects and delivers the lymph fluid from the distal part to proximal ALN to maintain the flow of lymph, the efficacy of the artificial structure is evaluated by confirming whether this condition is maintained even after implantation.

### Example 1. Manufacture and implantation of artificial structure for preventing or treating lymphedema

As shown in FIG. 3, an artificial structure for preventing or treating lymphedema was manufactured. Specifically, PDMS and a curing agent were mixed at a ratio of 12:1 and poured flat into a 100-pi dish with a thickness of 500 µm. In an embodiment, the PDMS and the curing agent may be mixed at a ratio of 9:1 to 10:1, 10:1 to 11:1, or 11:1 to 12:1. The mixture was then cured in a vacuum chamber while removing air bubbles therefrom. After 12 hours of curing, cylinder molds with a diameter smaller than 250 µm were placed on the **PDMS at** regular intervals. Another **PDMS** mixture having the same mixing ratio with the curing agent was poured onto the cylinder molds to a thickness of 500 µm. After 24 hours of curing in a vacuum chamber, the cured cylinder molds were removed to complete the manufacture of an artificial structure. An image of the artificial structure manufactured as a sheet type is shown in FIG. 4. **The** manufactured sheet-type artificial structure can be used by cutting it to fit the size and length of a lymph node that has been removed during lymphadenectomy.

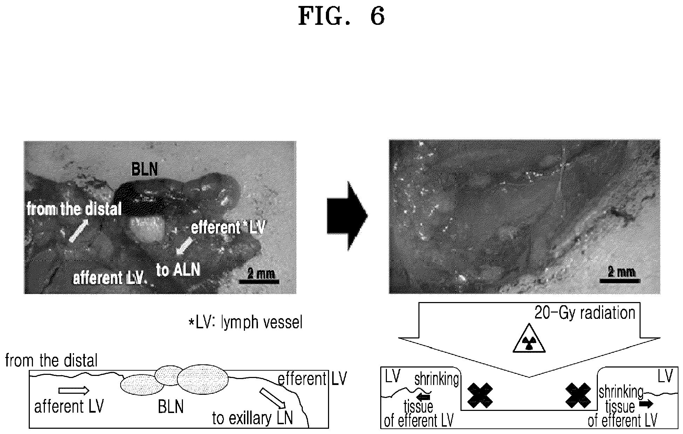

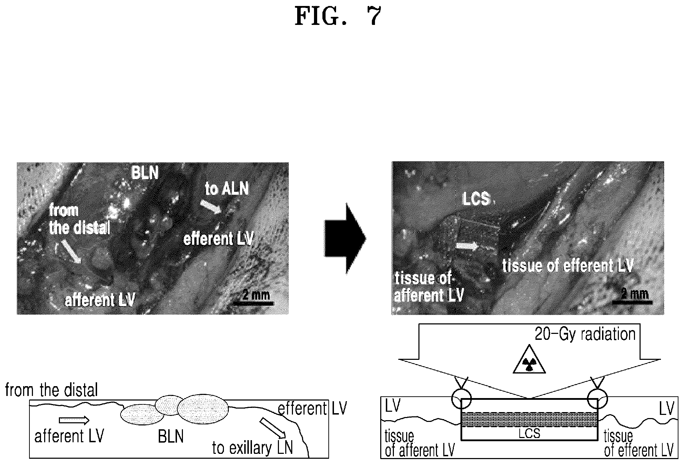

To implant the manufactured artificial structure in rats, 26 rats (JA BIO Inc., Republic of Korea) to be subjected to brachial lymph node (BLN) resection underwent skin incision that separates the distal part and the proximal part of the upper limb after the BLN resection, as shown in FIG. 5. As shown in FIG. 5, a situation of lymphadenectomy was stimulated by completely disconnecting the lymphatic connection through folding suture (a suturing technique of folding the skin and fixing the edges of the folds together with sutures, so that the lymphatic connection in the epidermis is blocked as the outer skin is connected to each other and therefore subsequently regenerating lymph cannot be connected to each other) or suture spacing (a suturing technique of placing the ends of skin incision 2 to 3 mm apart and fixing them to the muscles, so that the connection of lymph is blocked as a lesion where the lymph cannot be regenerated is developed between the skin). Next, 2 days after surgery and after sufficient recovery of the rats, 20-Gy of radiation was irradiated to the BLN dissection site to simulate a situation similar to radiotherapy and induce tissue fibrosis at the dissection site. Specifically, referring to FIGS. 6 and 7, secondary lymphedema was induced by leaving the dissected lymph node intact in a control group, whereas a lymphatic artificial structure was cut to the size of the lymph node at the site of the dissected lymph node and the inlet parts on both sides of the structure were connected by implantation using sutures on the cross-sections of the tissue each containing the disconnected lymphatic vessels in an experimental group. The rats were respiratory anaesthetized with 4 % isoflurane gas before surgery or follow-up examination, and the rats were further anaesthetized, during surgery, an intraperitoneal infusion of tiletamine/zolazepam (10 ml/kg; Zoletil, Virbac, France) mixed with xylazine (Rumpun, Bayer Korea, Republic of Korea) (volume ratio 5:1).

### Experimental Example 1. Evaluation of efficacy of artificial structure for preventing or treating lymphedema

Changes in an experimental group (n=13) implanted with the artificial structure and a control group (n=13) without implantation of the artificial structure were followed up for 8 weeks at the same time after lymphadenectomy and radiation exposure were performed as described in the previous example.

### 1.1.Follow-up for volume changes

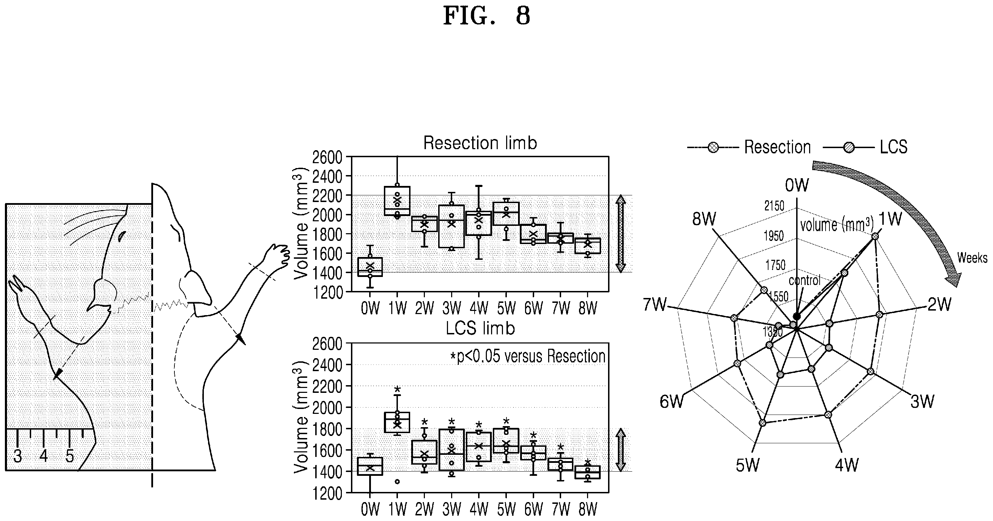

The rats in the control group and the experimental group were followed up weekly for 8 weeks after implantation. Here, the rats were fixed in a constant posture, and the length of each site was measured by using the ImageJ program from the visible light images obtained by a fixed stand imaging device, and the measured date were used to calculate the volume by the cone approximations. To determine whether edema was caused by lymph flow after lymphadenectomy and radiation exposure, the radius of the wrist base (distal part) of the rat and the distance to the baseline therefrom and the radius of the line (proximal part) connecting the elbow to fossa cubitalis of the rat and the distance to the baseline therefrom were measured with an imaging device to convert the volume, and the results are shown in FIG. 8.

As shown in FIG. 8, during the 8-week follow-up period, the experimental group showed a 50 % reduction in edema compared to the control group, and particularly at Week 5 and thereafter, edema was reduced to almost normal (rats not undergoing lymphadenectomy and radiation exposure).

### 1.2.Confirmation of connection of lymph flow

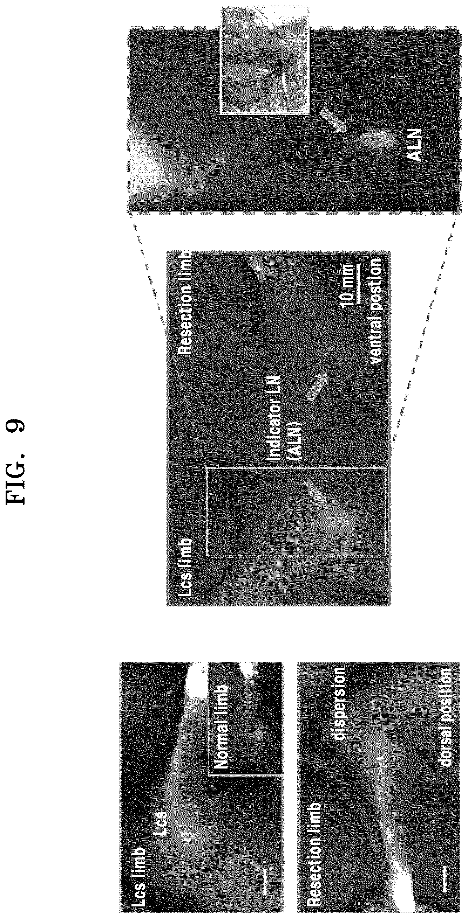

The rats of the control group and the experimental group were followed up weekly for 8 weeks after implantation. For fluorescence imaging of the lymphatic system, an indocyanine green (ICG) fluorescent substance was injected subcutaneously between toes and allowed to infiltrate through the microlymphatic vessels. Lymphography images were obtained by using a self-produced 730 nm light source and a camera for near-infrared (NIR) imaging. Since the BLN is anatomically connected directly to the axillary lymph node (ALN), the distally injected label substance is expected to travel past the resected BLN and be found in the ALN if the artificial structure bridges the disconnected lymph flow, ICG near-infrared lymphography was performed for follow-up. The ALN can be used as an indicator of the lymph flow, and every week, a fluorescent labeling substance was injected distally and indicators were irradiated to confirm lymphatic connection to the distal part, and the results are shown in FIG. 9. As shown in FIG. 9, the indicator lymph nodes could be identified, whereas the fluorescent contrast agent did not migrate to the indicator in the control group.

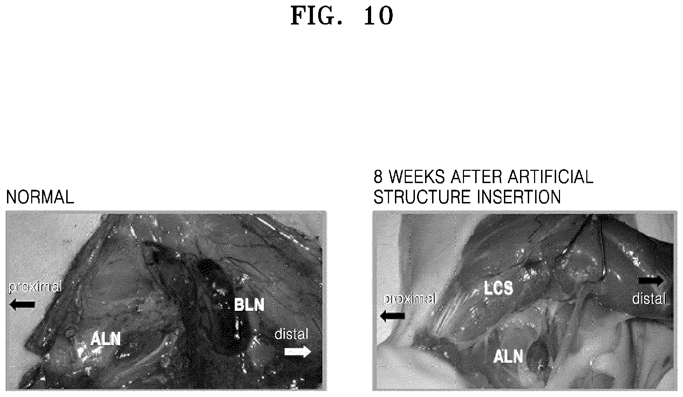

FIG. 10 shows photographs of a BLN and an ALN injected with a staining substance, Evans Blue, in the normal rats (rats not undergoing lymphadenectomy and radiation exposure) and in the rats 8 weeks after implantation of the artificial structure (i.e., LSC) that have been implanted.

As shown in FIG. 10, despite the effects of lymphadenectomy and radiation exposure, the flow of lymph fluid was maintained similar to normal by the implant.

### 1.3.Confirmation of lymphatic drainage pattern

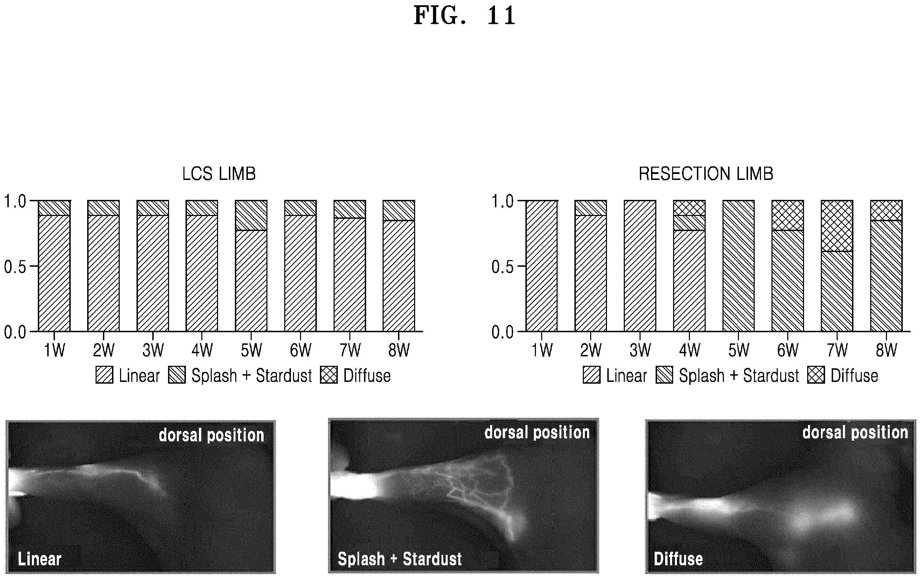

The rats in the control group and the experimental group were followed up weekly for 8 weeks after implantation. For fluorescence imaging of the lymphatic system, an ICG fluorescent substance was injected subcutaneously between toes and allowed to infiltrate through the microlymphatic vessels. Lymphography images were obtained by using a self-produced 730 nm light source and a camera for NIR imaging. When lymphography is applied to a patient, the opinion of normal lymph flow shows a linear pattern, whereas the opinion of a case where dermal backflow occurred due to lymphatic circulation disorder shows splash, stardust, diffuse patterns as the edema worsens, indicating the occurrence and development of lymphedema. Based on these opinions as a general-purpose standard, the lymphatic drainage pattern at the proximal site of the upper limb was examined, and the results are shown in FIG. 11.

As shown in FIG. 11, the experimental group continued to show a mostly linear lymphatic drainage pattern for 8 weeks, whereas the control group switched to show a pattern of dermal backflow after Week 5.

### 1.4.Confirmation of occurrence of lymphangiogenesis

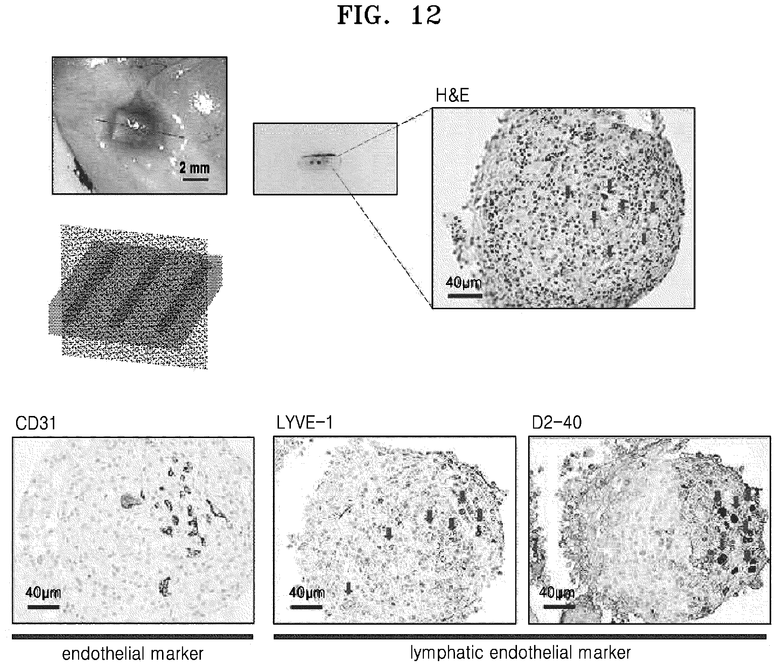

At Week 8 after implantation, histologic analysis was performed. Implants from the rats were extracted and cross sections thereof were stained with hematoxylin and eosin (H&E) to confirm the structure. By using an endothelial cell marker (CD31) and lymphatic endothelial cell markers (LYVE-1 and D2-40), lymphangiogenesis inside a channel was observed by immunohistochemistry (IHC). After 8 weeks of follow-up, the implanted lymphatic artificial structure was removed, and then, a cross section of the lymphatic channel inside the lymphatic artificial structure was examined by histology (H&E) and IHC to determine whether lymphangiogenesis occurs inside the channel, and the results are shown in FIG. 12.

As shown in FIG. 12, in the removed lymphatic artificial structure, structures that appeared to be cross-sections of channels were found along with tissue, and numerous lymphatic vessels and blood vessels were detected through the marker CD31, which stains specifically endothelial cells, and the markers LYVE-3 and D2-40, which stain specifically endothelial cells of lymphocytes, by H&E staining and IHC of cell nuclei.

### 1.5.Observation of changes in upper limb after removal of lymphatic artificial structure

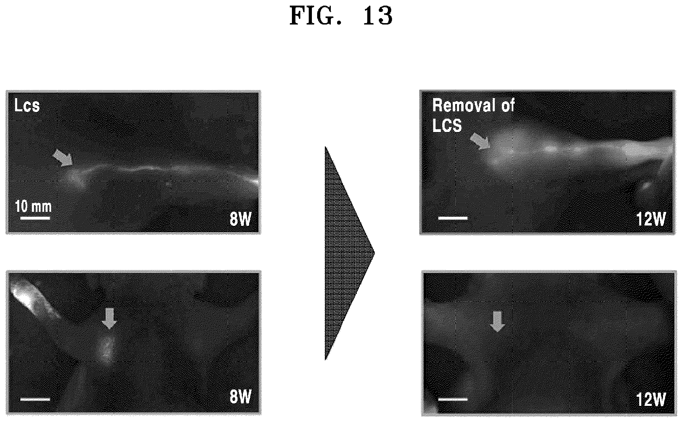

8 weeks after implantation, the implanted structure was removed, and through additional observation for 4 weeks, dependency of the lymph flow on artificial structure was confirmed. 4 weeks after the removal of the artificial structure, a fluorescent labeling agent was injected into the distal part in the same manner as in Experimental Example 1.2. Then, the lymphatic connection to the distal part was confirmed by examining the indicator, and the results are shown in FIG. 13.

As shown in FIG. 13, it is confirmed that there is normal lymph flow while the artificial structure is implanted, whereas the lymph fluid spreads near the lymphatic vessels and normal lymph flow is disrupted after removal of the artificial structure. Before removal of the artificial structure, the fluorescent labeling agent was found in the proximal ALNs. However, 4 weeks after removal of the artificial structure, the lymph flow was disrupted so that the fluorescent labeling agent could not be found in ALNs.

As a result of the foregoing, it was confirmed that the artificial structure according to an embodiment maintains the flow and drainage pattern of lymph similar to normal and has an effect on lymphangiogenesis after implantation, and the dependency of the lymph flow on the artificial structure was also confirmed. This means that the present disclosure can be used in the prevention or treatment of lymphedema.

### Explanation Of Reference Numerals Designating The Major Elements Of The Drawings

1: First sheet
2: Second sheet
10: Channel
100: Artificial structure
110: Inlet
120: Outlet
210: Primary channel
220: Anti-backflow channel

## Claims

1. An artificial structure comprising:
a first sheet; and
a second sheet arranged on the first sheet and including at least two channels, each of which includes an inlet and an outlet and is patterned on a first surface facing the first sheet, wherein the at least two channels each have a one-way channel structure that maintains a one-way flow of lymph from the inlet toward the outlet.

2. The artificial structure of claim 1, wherein the first sheet and the second sheet are prepared from a biocompatible polymer.

3. The artificial structure of claim 2, wherein the biocompatible polymer is any one selected from the group consisting of polylactic acid (PLA), polyglycolic acid (PGA), poly-ε-caprolactone (PCL), poly-L-lactic acid (PLLA), a poly(lactide-co-glycolic acid) (PLGA) copolymer, a poly(lactide-caprolactone (PLCL) copolymer, biodegradable polycarbonate, and a copolymer thereof.

4. The artificial structure of claim 2, wherein the biocompatible polymer is hydrophobic.

5. The artificial structure of claim 1, wherein the artificial structure constricts the at least two channels by the skeletal muscle pressure at an implanted site.

6. The artificial structure of claim 1, wherein the artificial structure forms a lymphatic network.

7. The artificial structure of claim 1, wherein the at least two channels each have a diameter of 200 to 1,000 µm.

8. The artificial structure of claim 1, wherein the at least two channels
each comprise:
a primary channel connecting the inlet to the outlet and providing a direction of main flow of lymph; and
at least two anti-backflow channels, each of which is connected to the primary channel and has a structure that prevents backflow.

9. The artificial structure of claim 8, wherein the primary channel has a zig-zagged linear structure, and
the at least two anti-backflow channels each have a curved structure.

10. The artificial structure of claim 9, wherein the at least two anti-backflow channels are staggered at regular intervals with respect to the primary channel.

11. The artificial structure of claims 1 to 10, wherein the artificial structure is implanted in any one of a branchial lymph node (BLN), a superficial inguinal node, a deep inguinal node, and a popliteal lymph node.

12. The artificial structure of claims 1 to 10, wherein the artificial structure is implanted in a branchial lymph node (BLN).

13. A construction method for an artificial structure, wherein the artificial structure comprises: a first sheet; and a second sheet arranged on the first sheet and including at least two channels, each of which includes an inlet and an outlet and is patterned on a first surface facing the first sheet, wherein the at least two channels each have a one-way channel structure that maintains a one-way flow of lymph from the inlet toward the outlet, the construction method comprising:
preparing the first sheet and the second sheet that are prepared from a polymer; and
adhering the first sheet and the second sheet to position patterning on the first side of the second sheet to face inwardly.

14. The construction method of claim 13, wherein the polymer is polydimethylsiloxane (PDMS).

15. The construction method of claim 13, wherein the artificial structure is prepared from a biocompatible polymer.

16. The construction method of claim 15, wherein biocompatible polymer is any one selected from the group consisting of polylactic acid (PLA), polyglycolic acid (PGA), poly-ε-caprolactone (PCL), poly-L-lactic acid (PLLA), a poly(lactide-co-glycolic acid) (PLGA) copolymer, a poly(lactide-caprolactone (PLCL) copolymer, biodegradable polycarbonate, and a copolymer thereof.

17. The construction method of claim 15, wherein the biocompatible polymer is hydrophobic.
